# EUROPÄISCHE PATENTSCHRIFT

(11) **EP 1 670 423 B1**
(45) Veröffentlichungstag und Bekanntmachung des Hinweises auf die Patenterteilung: **08.10.2008**
(21) Anmeldenummer: 04730839.0
(22) Anmeldetag: 03.05.2004
(51) Int. Cl.: A61K 8/04, A61K 8/34, A61K 8/41, A61Q 5/10

(54) **FARBSTOFFHALTIGE PELLETS ZUM FÄRBEN VON KERATINFASERN**
DYE-CONTAINING PELLETS FOR DYEING KERATIN FIBRES
PASTILLES RENFERMANT UN COLORANT POUR LA COLORATION DES FIBRES KERATINIQUES

(30) Priorität: 10.10.2003 DE 10347242
(43) Veröffentlichungstag der Anmeldung: 21.06.2006
(73) Patentinhaber: Wella Aktiengesellschaft, 64274 Darmstadt (DE)
(72) Erfinder: SCHMENGER Jürgen, 64331 Weiterstadt (DE); BRAUN, Petra, 64839 Münster (DE); ENGLISCH, Wolfram, 01728 Bannewitz (DE)
(74) Vertreter: Bockhorst, Matthias
(86) Internationale Anmeldenummer: PCT/EP2004/004632
(87) Internationale Veröffentlichungsnummer: WO 2005/044208

(56) Entgegenhaltungen:
- EP-A- 0 322 461
- EP-A- 0 525 389
- EP-A- 0 630 643
- EP-A- 0 689 867
- WO-A-99/42087
- DE-A- 4 233 874
- DE-C- 3 910 275
- GB-A- 576 100
- GB-A- 1 086 150
- MÄGDEFESSEL-HERMANN, K.: "Frischer Wind im Produkt-Design (Wirbelschicht-Coating, Techniktrend)" CHEMIE PRODUKTION, [Online] Nr. 9, 2002, XP002293838 HOME > PRESSE > PRESSESPIEGEL (FA. GLATT) Gefunden im Internet: URL:http://www.glatt.de/d/11_presse/11_03_ 11.htm> [gefunden am 2004-08-25]
- DATABASE WPI Section Ch, Week 197342 Derwent Publications Ltd., London, GB; Class D21, AN 1973-62612U XP002293756 & JP 48 049935 A (HO-E-CO LTD) 14. Juli 1973 (1973-07-14)
- "Eintrag zu Pellets" 1985, RÖMPPS CHEMIE-LEXIKON

## Beschreibung

Gegenstand der vorliegenden Erfindung ist ein Verfahren zur Herstellung farbstoffhaltiger Pellets, sowie die Verwendung dieser Pellets zur Färbung von Keratinfasern.

Farbstoffe, die üblicherweise für die Färbung von Keratinfasern eingesetzt werden, sind sogenannte Direktzieher, Nitrofarbstoffe und Pigmentfarbstoffe oder Oxidationsfarbstoffe, die als farblose Entwickler-/Kupplervorstufen vorliegen.

Nach dem Stand der Technik hergestellte Färbemittel werden in üblichen Darreichungsformen angeboten. Diese Darreichungsformen variieren von flüssigen bis zu creme- und wachsartigen Produkten. Auch Aerosole, beispielsweise sogenannte Schaumhaarfarben, finden Anwendung. Ebenso sind nach dem Stand der Technik pulverförmige Farben auf dem Markt, die vor der Anwendung mit einem wässrigen Medium gemischt werden müssen.
Im Stand der Technik EP 0 630 643 A1, DE 42 33 874 A1 und EP 0 689 867 A1 werden auch Granulate beschrieben, welche sich jedoch von den erfindungsgemäßen Pellets unterscheiden.

Die vorgenannten Mittel sind jedoch nicht in jeder Hinsicht befriedigend So wird zum Beispiel bei staubfreien, ölversetzten Pulvern durch die eingesetzten Öle die Produkt leistung beeinträchtigt, während bei Einsatz von reaktiven Farbstoffen und Rohstoffen sowohl bei Pulvern als auch bei flüssigen Systemen Probleme im Hinblick auf die Lagerstabilität auftreten.

Überraschenderweise konnte mit Hilfe eines geeigneten Verfahrens, welches unter Zuhilfenahme geeigneter Träger- und Verkapselungsmaterialen (Coating) durchgeführt wird, ein farbstoff haltiges Pellet zum Färben keratinischer Fasern entwickelt werden, welches die oben beschriebenen Nachteile nicht aufweist und zudem eine bessere Dosierbarkeit sowie Multicolor-Effekte ermöglicht.

Gegenstand der vorliegenden Anmeldung ist daher ein Verfahren zur Herstellung farbstoffhaltiger Pellets, welches dadurch gekennzeichnet ist, dass a) in einem Vertikalgranullerer bei einer Rotordrehzahl von 50 bis 200 U/min, (vorzugsweise etwa 150 U/min) einer Zerhackerdrehzahl von 750 bis 1250 U/min (vorzugsweise etwa 1000 U/min) und einer Temperatur von 15 bis 35 °C. durch Trockenmischen und anschließendes Nassmischen einer mindestens eine Oxidationsfarbstoffvorstufe enthaltenden Farbstoffmasse mit einem Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffen eine Grundmasse hergestellt wird; b) anschließend diese Grundmasse in einem Extruder bei einer Drehzahl von 15 bis 50 U/min (vorzugsweise etwa 25 bis 30 U/min) und einer Lochgröße der Siebe von etwa 0,01 bis 5 mm (vorzugsweise etwa 0,6 bis 1 mm) extrudiert wird; c) das so erhaltene Granulat in einem Pelletizer bei einer Drehzahl von 400 bis 800 U/min (vorzugsweise 500 bis 600 U/min) verrundet wird; d) das Granulat sodann bei einer Produkttemperatur von 20 bis 60 °C (vorzugsweise 30 bis 55°C) und einer Zulufttemperatur von 70 bis 80 °C getrocknet wird; e) anschließend (ggfs nach vorherigem Erwärmen auf 40-50°C) das Granulat bei einer Sprührate von 5 bis 20 g/min und einem Sprühluftdruck von 1,5 bis 2,5 bar im Wirbelschichtverfahren gecoatet wird, wobei das Verkapsälungsmaterial, welches ausgewählt ist aus Cellulose-Derivaten, Polyethylen-Dispersionen, Polyacrylsäuren, Polyvinylalkoholen, Polyvinylpyrrplidonen, Polycarbonaten, Polyestern, Polyamiden und natürlichen Filmbildnern, -bezogen auf die Menge des zu coatenden Granulates- in einer Menge von 0,5 bis 50 Gewichtsprozent (vorzugsweise 1 bis 20 gew.%, insbesondere 2 bis 15 gew.%) eingesetzt wird, und f) das Produkt abschließend bei einer Produkttemperatur von maximal 51 °C getrocknet wird.

Ein weiterer gegenstand der vorliegenden Anmeldung ist ein Verfahren zur Herstellung farbstoffhaltiger Pellets, welches dadurch gekennzeichnet ist, daß

in einem Wirbelschichtgranulierer coater a) bei einer Rotordrehzahl von 50 bis 200 U/min (vorzugsweise etwa 150 U/min), einer Zerhackerdrehzahl von 750 bis 1250 U/min (vorzugsweise etwa 1000 U/min) und einer Temperatur von 15 bis 35 °C eine mindestens eine Oxidationsfarbstoffvorstufe enthaltende Farbstoffmasse mit einem Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffen vermischt wird, anschließend auf eine Temperatur von max. 34 °C temperiert und sodann granuliert wird; b) das erhaltene Granulat bei einer Sprührate von 6 bis 20 g/min und einem Sprühluftdruck von 0,25 bis 0,75 bar mit einem Verkapselungsmaterial, welches ausgewählt ist aus Cellulose-Derivaten, Polyethylen-Dispersionen, Polyacrylsäuren, Polyvinylalkoholen, Polyvinylpyrrplidonen, Polycarbonaten, Polyester, Polyamiden und natürlichen Filmbildnern gecoatet wird, wobei das Verkapselungsmaterial -bezogen auf die Menge des zu coatenden Granulates- in einer Menge von 0,5 bis 50 Gewichtsprozent (vorzugsweise 1 bis 20 gew.% und insbesondere 2 bis 10 gew.%) eingesetzt wird; und c) falls erforderlich, das erhaltene Produkt bei einer Produkttemperatur von max. 60 °C getrocknet wird.

Ein weiterer gegenstand der vorliegenden Anmeldung ist ein Verfahren zur Herstellung farbstoffhaltiger Pellets, welches dadurch gekennzeichnet ist,
dass in einem Wirbelschichtgranulierer coater a) bei einer Rotordrehzahl von 50 bis 200 U/mim vorzugsweise etwa 150 U/min einer Zerhackerdrehzahl von 750 bis 1250 U/min vorzugsweise etwa 1000 U/min und einer Temperatur von 15 bis 35 °C das Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffe miteinander vermischt werden; b) die so erhaltene Grundmasse auf eine Temperatur von max. 34 °C temperiert und granuliert wird; c) sodann bei einer Sprührate von 6 bis 20 g/min und einem Sprühluftdruck von 0,25 bis 0,75 bar mit einer Lösung oder Dispersion der mindestens eine Oxidationsfarbstoffvorstufe enthaltenden Farbstoffmasse in einem Verkapselungsmaterial, welches ausgewählt ist aus Cellulose-Derivaten. Polyethylen-Dispersionen, Polyacrylsäuren, Polyvinylalkoholen, Polyvinylpyrrplidonen, Polycarbonaten, Polyestern, Polyamiden und natürlichen Filmbildnern, gecoatet wird, wobei das Verkapselungsmaterial - bezogen auf die Menge des zu coatenden Granulates- in einer Menge von 0,5 bis 50 Gewichtsprozent vorzugsweise 1 bis 20 gew.% und insbesondere 2 bis 10 gew.% eingesetzt wird; und d) falls erforderlich, das erhaltene Produkt bei einer Produkttemperatur von max. 57 °C getrocknet wird.

Die vorgenannten Herstellungs verfahren sind in Anlage 1 (Extrudiertechnologie) oder Anlage 2 (Top-Spray-Verfahren) Schematisch dargestellt.

Geeignete Trägermaterialien sind Polyvinylpyrrolidon, Dextrose, Oligosaccharide, mikrokristalline Cellulose-Derivate, wie zum Beispiel Hydroxypropylmethylcellulose, Methylcellulose, Hydroxyethylcellulose, Hydroxypropylcellulose, Nonoxynol-Hydroxyethyl-cellulose und Cetyl-Hydroxyethylcellulose oder physikalisch beziehungsweise chemisch modifizierte Stärken oder Stärkederivate, wie zum Beispiel Stärkeester (beispielsweise acetylierte Stärken), Stärkeether (beispielsweise hydroxyalkylierte Stärken), Dialdehydstärken, Dicarboxylstärken, Distärkephosphate, Hydroxyalkylstärkephosphate oder Hydroxyalkylstärken, wobei die Alkylgruppen vorzugsweise 1 bis 4, besonders bevorzugt 2 bis 3 C-Atome enthalten. Geeignet sind auch quervernetzte Stärkeether, wie zum Beispiel solche mit den INCI-Bezeichnungen Dimethylimidazolidone Rice beziehungsweise Corn Starch oder hydrophob modifizierte Stärken (beispielsweise solche mit der INCI-Bezeichnung Aluminium Starch Octensuccinate). Die Stärke kann sowohl thermisch als auch hydrolytisch oder enzymatisch modifiziert worden sein, wobei die Ausgangsstärke aus den bekannten Quellen, beispielsweise Mais, Kartoffeln, Süsskartoffeln, Erbsen, Bananen, Hafer, Weizen, Gerste, Reis, Sago, Tapioca, Pfeilwurz, Amarant, Kanna, Sorghum, usw., gewonnen werden kann. Besonders bevorzugte Stärkederivate sind nichtionische Stärkederivate, insbesondere mit Alkylenoxiden wie Ethylenoxid, Propylenoxid oder Butylenoxid, Acetanhydrid oder Butylketendimer, und insbesondere Propylenoxid, modifizierte nichtionische Stärkederivate. Weitere geeignete Trägermaterialien sind synthetisches Calciumsilicat, Kieselgur, Siliziumdioxid.

Geeignete Verkapselungsmaterialien sind Cellulose-Derivate (beispielsweise Methylcellulosen), Polyethylen-Dispersionen, Polyacrylsäuren, Polyvinylalkohole, Polycarbonate, Polyvinylpyrrolidon, Polyester und Polyamide oder natürliche Filmbildner wie zum Beispiel Chitosan, Schellack, Oligosaccharide oder auch chinesisches Balsamharz (Kolophonium).

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-pheno, 4-Amino-2-(2-hydroxyethyl)-phenol, 5-Amino-salicylsäüre, 2,5-Diamino-pyridin, 2,4,5,6-Tetraamino-pyrimidin, 2,5,6-Triamino-4-(1 H)-pyrimidon, 4,5-Diamino-1 -(2-hydroxyethyl)-1 H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-diamino-1 H-pyrazol, 4,5-Diamino-1-methyl-1H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol; 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Unter den vorgenannten Oxidationsfarbstoffen sind die folgenden Verbindungen, alleine oder in Kombination miteinander besonders bevorzugt: 2,5-Diamino-toluol, 2,4-Diaminophenoxyethanol, Resorcin, 2-Methylresorcin, m-Aminophenol, 4-Amino-m-kresol, 4-Amino-2-hydroxytoluol, 6-Amino-m-kresol, 2-Amino-4.-hydroxyethylaminoanisol, 1-Naphthol, Hydroxyethyl-3,4-methylendioxyanilin, 2,5-Diamino-phenylethanol, N,N-Bis(2-hydroxyethyl)-p-phenylendiamin, Phenyl-methyl-pyrazolon, 1-Hydroxyethyl-4,5-diamino-pyrazol und 2-Amino-6-chlor-4-nitro-phenol oder deren Salze.

Die Gesamtmenge der in den Pellets enthaltenen Oxidationsfarbstoffvorstufen beträgt etwa 0,1 bis 70 Gewichtsprozent, insbesondere etwa 0,5 bis 50 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo,Triphenylmethanfarbstoffe, aromatische Nitrofarbstoffe, Azofarbstofte, Chinonfarbstoffe, kationische oder anionische Farbstoffe, zugesetzt werden.

Als geeignete synthetische Farbstoffe können beispielsweise genannt werden: Hydroxyethyl-2-nitro-p-toluidin, 2-Hydroxyethyl-pikraminsäure, 4-Nitrophenyl-aminoharnstoff, Tri(4-amino-3-methylphenyl)-carbeniumchlorid (Basic Violet 2), 1,4-Diamino-9,10-anthracendion (Disperse Violet 1), 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitrodiphenylamin (HC Red No. 1), 1 -Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methyl-amino-4-nitrobenzol, 2-[(2-Hydroxyethyl)amino]-5-nitrophenol (HC Yellow No. 11), 3-[(2-Aminoethyl)-amino]-1-methoxy-4.-nitrobenzol-hydrochlorid (HC Yellow No.9), 1-[(2-Ureidoethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethylhamino]-3-nitro-1-trifluormethyl-benzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)amino]-9,10-anthrachinon, 1-[(2-Hydroxy-ethyl)amino]-4-methyl-amino-9,10-anthrachinon (Cl61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)-amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500 Disperse Blue No. 7, Solvent Blue No. 69), 9-(Dimethylamino)-benzo[a]-phenoxazin-7-ium-chlorid (Cl51175; Basic Blue No. 6), Di[4-(diethyl-amino)phenyl][4-(ethylamino)naphthyl]-carbenium-chlorid (Cl42595; Basic Blue No. 7), 3,7-Di(dimethylamino)-phenothiazin-5-iumchlorid (Cl52015; Basic Blue No. 9), Di[4-(dimethyl-amino)phenyl][4-(phenylamino)naphthyl]-carbenium-chlorid (Cl44045; Basic Blue No. 26), 2-[(4-(Ethyl(2-hydroxy-ethyl)amino)phenyl)azo]-6-methoxy-3-methylbenzothiazolium-methylsulfat (Cl11154; Basic Blue No. 41), 8-Amino-2-brom-5-hydroxy-4-imino-6-[(3-(trimethylammonio)phenyl)-amino]-1 (4H)-naphthalinon-chlorid (Cl56059; Basic Blue No. 99), Bis[4-(dimethylamino)phenyl][4-(methyl-amino)phenyl]carbenium-chlorid (Cl42535; Basic Violet No. 1), Tris[4-(dimethylamino)phenyl]carbenium-chlorid (Cl42555 Basic Violet No. 3), 2-[3,6-(Diethylamino)-dibenzopyranium-9-yl]-benzoesäure-chlorid (Cl45170; Basic Violet No. 10), Di(4-aminophenyl)-(4-amino-3-methyl-phenyl)carbenium-chlorid (Cl42510; Basic Violet No. 14), 1,3-Bis[(2,4-diamino-5-methylphenyl)azo]-3-methylbenzol (Cl21010; Basic Brown No. 4), 1-[(4-Aminophenyl)azo]-7-(trimethylammonio)-2-naphthol-chlorid (Cl12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphtholchlorid (Cl12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)-azo]-1,2,4-triazolium-chlorid (Cl11055; Basic Red No. 22), 2-Hydroxy-1-[(2-methoxy-phenyl)azo]-7-(trimethylammonio)-naphthalin-chlorid (Cl12245; Basic Red No. 76), 2-[2-((2,4-Dimethoxyphenyl)amino)ethenyl]-1,3,3-trimethyl-3H-indol-1-ium-chlorid (Cl48055 Basic Yellow No. 11), 3-Methyl-1-phenyl-4-[(3-(trimethylammonio)-phenyl)azo]-pyrazol-5-on-chlorid (Cl12719; Basic Yellow No. 57), Bis[4-(diethylamino)phenyl]phenylcarbenium-hydrogensulfat(1:1) (Cl42040; Basic Green No. 1), 1-[Di(2-hydroxyethyl)-amino]-3-methyl-4-[(4-nitro-phenyl)azo]-benzol (Cl11210, Disperse Red No. 17), 4-[(4-Aminophenyl)-azo]-1-[di(2-hydroxyethyl_{.})amino]-3-methyl-benzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)-amino]-2-phenylamino-benzolsulfonsäure-natriumsalz (Cl10385; Acid Orange No. 3), 4-[(2,4-Dihydroxyphenyl)aio]-benzolsulfonsäuremononatriumsalz (Cl14270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2, 4-naphthalindisulfonsäure-trinatriumsalz (Cl16255; Ponceau 4R; Acid Red No. 18), 3-Hydroxy-4-[(4-sulfonaphth-1-yl)azo]-2,7-naphthalin-disulfonsäuretrinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäuredinatriumsalz (Cl45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (Cl45100; Acid Red No. 52), 8-[(4-(Phenylazo)-phenyl)azo]-7-naphthol-1,3-disulfonsäure-dinatriumsalz (Cl27290; Acid Red No. 73), 2',4',5',7'-Tetrabrom-3',6'-dihydroxyspiro-[isobenzofuran-1 (3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (Cl45410; Acid Red No. 92), 3',6'-Dihydroxy-4',5'-diiodospiro[isobenzofuran-1 (3H),9'(9H)-xanthen]-3-on-dinatriumsalz (Cl45425; Acid Red No. 95), (2-Sulfophenyl)di[4-(ethyl((4-sulfophenyl)methyl)amino)phenyl]-carbenium-dinatriumsalz, betain (C142090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxynaphth-1-yl)carbenium-inneres Salz, mononatriumsalz (Cl44090; Food Green No. 4; Acid Green No. 50), Bis[4-(diethylamino)phenyl](2,4-disulfophenyl)carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (Cl42051; Acid Blue No. 3), 1-Amino-4-(cyclohexylamino)-9,10-anthrachinon-2-sulfonsäure-natriumsalz (Cl62045; Acid Blue No. 62),2-(1,3-Dihydro-3-oxo-5-sulfo-2H-indol-2-yliden)-2,3-dihydro-3-oxo-1H-indol-5-sulfonsäuredinatriumsalz (Cl73015; Acid Blue No. 74), 9-(2-Carboxyphenyl)-3-[(2-methylphenyl)amino]-6-[(2-methyl-4.-sulfophenyl)amino]xanthylium-inneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalindisulfonsäure-dinatriumsalz (Cl20470; Acid Black No. 1), 3-Hydroxy-4-[(2-hydroxynaphth-1-yl)azo]-7-nitro-1-naphthalin-sulfonsäurechromkomplex (3:2) (Cl15711 ; Acid Black No. 52), 3-[(2,4-Dimethyl-5-sulfophenyl)azo]-4-hydroxy-1-naphthalin-sulfonsäure-dinatriumsalz (Cl14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (Cl28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), 3',3",4,5,5',5",6,7-Octabromphenolsulfonphtalein (Tetrabromphenolblue), 1-((4-Amino-3,5-dimethylphenyl)-(2,6-dichlorphenyl)methylen)-3,5-dimethyl-4-imino-2,5-Cyclohexadien-verb mit Phosphorsäure(1:1) (Basic Blue 77), 2',4',5',7'-tetrabrom-4,5,6,7-tetrachlor-3',6'-dihydroxy-Spiro[isobenzofuran-1 (3H),9'[9H]xanthen]-3-on-dinatriumsalz (Acid Red 92), N,N-Di(2-hydroxyethyl)-3-methyl-4-((4-nitrophenyl)azo)-anilin (Disperse Red 17), 2,4-Dinitro-1-naphthol-7-sulfonsäure-dinatriumsalz (Acid Yellow 1), 4-((2-Hydroxynaphthalin-1-yl)azo)-benzolsulfonsäure-natriumsalz (Acid Orange 7), 2-((4-(Ethyl(2-hydroxyethyl)amino)-2-methylphenyl)azo)-5-nitro-1,3-thiazol (Disperse Blue 106), 2,4-Dinitro-1-naphtol, 2-[(4-Aminophenyl)-azo]-1,3-dimethyl-1H-imidazo-3-lium-chlorid, 1-Methyl-4-((methylphenylhydrazono)methyl)-pyridiniummethosulfat, 2-[[4-(Dimethylamino)phenyl]-azo]-1,3-dimethyl imidazolium-chlorid, 2-((4-((4-Methoxyphenyl)aminophenyl)azo)-1,3-dimethyl-1H-Imidazol-3-ium-chlorid und 1,3-Dimethyl-2-((4-((phenylmethyl)amino) phenyl)azo)-1H-Imidazol-3-ium-chlorid, alleine oder in Kombination miteinander.

Unter den vorgenannten direktziehenden Farbstoffen sind die folgenden Verbindungen -alleine oder in Kombination miteinander- besonders bevorzugt: Hydroxyethyl-2-nitro-p-toluidin, 2-Hydroxyethyl-pikraminsäure, 4-Nitrophenyl-aminoharnstoff, Tri(4-amino-3-methylphenyl)-carbeniumchlorid (Basic Violet 2), 1,4-Diamino-9,10-anthracendion (Disperse Violet 1), 1-(2-Hydroxyethyl)-amino-2-nitro-4-[di(2-hydroxyethyl)-amino]-benzol (HC Blue No. 2), 4-[Ethyl-(2-hydroxyethyl)amino]-1-[(2-hydroxyethyl)-amino]-2-nitro-benzol-hydrochlorid (HC Blue No. 12), 1-Amino-4-[di(2-hydroxyethyl)amino]-2-nitrobenzol-hydrochlorid (HC Red No. 13), 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-Chlor-6-ethylamino-4-nitrophenol, 2-Amino-6-chlor-4-nitrophenol, 4-[(2-Hydroxyethyl)-amino]-3-nitro-1-trifluormethyl-benzol (HC Yellow No. 13), 8-Amino-2-brom-5-hydroxy-4-imino-6-[(3-(trimethylammonio)phenyl)-amino]-1 (4H)-naphthalinon-chlorid (C156059; Basic Blue No. 99), 1-[(4-Aminophenyl)azo]-7-(trimethylammonio)-2-naphthol-chlorid (Cl12250; Basic Brown No. 16), 1-[(4-Amino-2-nitrophenyl)azo]-7-(trimethylammonio)-2-naphthol-chlorid (Basic Brown No. 17), 2-Hydroxy-1-[(2-methoxy-phenyl)azo]-7-(trimethylammonio)-naphthalin-chlorid (Cl12245; Basic Red No. 76), 3-Methyl-1-phenyl-4-[(3-(trimethylammonio)-phenyl)azo]-pyrazol-5-on-chlorid (Cl12719; Basic Yellow No. 57) und 2,6-Diamino-3-[(pyridin-3-yl)azo]-pyridin sowie deren Salze.

Die Gesamtmenge der direktziehenden Farbstoffe beträgt in den Pellets etwa 0,1 bis 90 Gewichtsprozent, vorzugsweise 1 bis 70 Gewichtsprozent.

Weitere für die Verwendung in Haarfärbemitteln bekannte und übliche Farbstoffe sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben, auf die heirmit ausdrücklich Bezug genommen wird.

Die nach dem erfindungsgemäßen Verfahren hergestellten farbstoffhaltigen Pellets weisen eine Vielzahl von Vorteilen auf. So sind sie absolut staubfrei, wobei die Nachteile von üblichen staubfreien, ölversetzten Pulvern - insbesondere die Beeinträchtigung der Produktleistung- nicht auftreten. Je nach Art und Schichtdicke des gewählten Coatingmaterials ist eine Freisetzung der Farbstoffe zu einem beliebig wählbaren Zeitpunkt möglich (retardierte Freisetzung). Ebenfalls ist der gemeinsame Einsatz von reaktiven Farbstoffen und Rohstoffen (z.B. Oxidationsmitteln wie Persulfaten und Wasserstoffperoxidsalzen oder Wasserstoffperoxidaddukten) sowie eine deutlich verbesserte Lagerstabilität gegenüber Pulvern und wässrig/alkoholischen Systemen möglich. Besonders interessant ist die Möglichkeit der unkomplizierten Erzielung von Multicolor-Effekten durch Art und Schichtdicke des gewählten Coatingmaterials, während nach dem heutigen Stand der Technik derartige Multicolor-Effekte nur durch extrem zeitaufwendige und komplizierte Techniken, wie beispielsweise Folientechniken oder Strähnentechniken, zu erzielen sind. Zudem weisen die Pellets eine im Vergleich zu Färbemitteln nach dem Stand der Technik (Pulver, wässrig/alkoholische Systeme) deutlich bessere Dosierbarkeit auf.

Ein weiterer Gegenstand der vorliegenden Erfindung ist daher die Verwendung der vorgenannten Pellets zur Herstellung von Färbemitteln für Keratinfasern sowie ein Mittel zur Färbung von Keratinfasern, wie zum Beispiel Haaren und insbesondere menschlichen Haaren, welches durch Vermischen der vorgenannten Pellets mit einer wässrigen oder wässrigalkoholischen Zubereitung hergestellt wird.

Als wässrige oder wässrig-alkoholische Zubereitung können sowohl Wasser oder ein Gemisch aus Wasser und C1-C6-Alkoholen (z.B. Ethanol oder Isopropanol) oder eine übliche Wasserstoffperoxidlösung oder Wasserstoffperoxidemulsion als auch ein übliches Haarreinigungsmittel, Haarkonditionierungsmittel oder Haarfestigungsmittel verwendet werden.

Die Zusammensetzung derartiger Zubereitung ist bekannt und kann den einschlägigen Kosmetiklehrbüchern, beispielsweise Karlheinz Schrader, "Grundlagen und Rezepturen der Kosmetika", 2. Auflage (1989), auf die hiermit ausdrücklich Bezug genommen wird, entnommen werden.

In dem erfindungsgemäßen Färbemittel können beispielsweise Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Weiterhin kann das erfindungsgemäße Färbemittel für derartige Mittel übliche Hilfs- und Zusatzstoffe, wie zum Beispiel Verdickungsmittel, beispielsweise Homopolymere der Acrylsäure, Pflanzen-Gums, Algenpolyasaccharide, amphiphile Assoziatiwerdicker, desweiteren Konservierungsstoffe; Komplexbildner; Netzmittel oder Emulgatoren aus den Klassen der anionischen, kationischen, amphoteren oder nichtionogenen oberflächenaktiven Substanzen; Alkalisierungsmittel (z.B. Ammoniumsalze oder Aminosäuren wie Glycin und Alanin); sowie Pflegestoffe, wie kationische Polymere oder Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Die Oxidationsfarbstoffvorstufen enthaltenden Färbemittel werden in Kombination mit einem oder mehreren bekannten chemischen Oxidationsmitteln, beispielsweise Wasserstoffperoxid oder dessen Salzen oder Addukten sowie Persulfaten wie Natriumpersulfat, Kaliumpersulfat oder Ammoniumpersulfat, verwendet, oder durch Luftoxidation (ggfs. in Gegenwart geeigneter Enzyme oder Katalysatoren) aktiviert.

Ebenso können die erfindungsgemäßen farbstoffhaltigen Pellets Ammoniumcarbonate wie beispielsweise Ammoniumhydrogencarbonat oder Aminosäuren und deren Salze wie beispielsweise Natriumglycinat enthalten, um so eine gleichzeitige Aufhellung der Faser zu erreichen.

Die nachfolgenden Beispiele sollen den Erfindungsgegenstand näher erläutern ohne diesen jedoch einzuschränken.

### Beispiele

### Beispiel 1: Herstellung von Farbstoffpellets im Top-Spray-Verfahren

In einem Glatt-Wirbelschichtgranulator und Coater wird die folgende Mischung A bei einer Zulufttemperatur von 90 °C und einer Luftmenge von 18 m³/h auf eine Produkttemperatur von 34 °C erwärmt.

| Mischung A | |
|---|---|
| 7,2 g | 5-Amino-2-methyl-phenol |
| 16,0 g | 2,5-Diamino-toluol-sulfat |
| 4,0 g | 2-Amino-4-(β-hydroxyethylamino)-anisol-sulfat |
| 3,0 g | Ascorbinsäure |
| 4,0 g | Natriumsulfit |
| 965,8 g | Hydrolyzed Corn Starch (Oligosaccharid) |

Anschließend wird eine 20 %ige wässrige Polyvinylpyrrolidon-Lösung ("Sprühlösung") mit einer Anfangs-Sprührate von 8 g/min und einem Sprühluftdruck von 0,5 bar auf diese Mischung aufgesprüht. Im Verlauf des Granulierprozesses werden die Sprührate auf 12 g/min und die Zulufttemperatur auf 100 °C erhöht, wobei die Luftmenge auf max. 30 m³/h gesteigert wird. Die Produkttemperatur wird während des gesamten Verfahrens auf etwa 30-31 °C gehalten. Nach dem Auftrag von 563 g werden die Pellets bei einer max. Produkttemperatur von 57 °C getrocknet, anschließend auf etwa 30 °C abgekühlt und gesiebt.

**Beispiel 2: Herstellung von Farbstoffpellets mittels Extrudiertechnologie**

| Mischung A | |
|---|---|
| 1411 g | 2,5-Diamino-toluol-sulfat |
| 636 g | 4-Amino-2-hydroxytoluol |
| 353 g | 2-Amino-4-(β-hydroxyethylamino)-anisol-sulfat |
| 794 g | Ascorbinsäure |
| 1058 g | Natriumsulfit |
| 800 g | Hydroxypropylcellulose |
| 1300 g | Maisstärke |

Die Mischung A wird in einem Vertikalgranulierer (Rotordrehzahl = etwa 150 U/min; Zerhackerdrehzahl = etwa 1000 U/min) 1 Minute lang vermischt und anschließend mittels einer Zweistoffdüse unter weiterem Mischen mit 2091 g einer 5,625 %ige wässrige Hydroxypropyl-methylcellulose-Lösung besprüht. Die so erhaltene Masse wird mittels eines Extruders Typ BR 200 (Drehzahl = 27 U/min; SiebØ: 1,0 mm) bei einer Produkttemperatur von etwa 30 °C extrudiert. Anschließend wird das so erhaltene Material in einem Pelletizer Typ P 50 1 Minute lang bei 550 U/min verrundet und sodann in einem Glatt-Vertikalgranulierer bei einer Zulufttemperatur von 70 °C und einer Luftmenge von etwa 60-90 m³/h sowie einer max. Produkttemperatur von 51 °C getrocknet.
In einem Glatt-Wirbelschichtgranulator und Coater werden 1500 g der getrockneten Farbstoffpellets bei einer Zulufttemperatur von etwa 50 °C und einer Luftmenge von 75 m³/h auf eine Produkttemperatur von 39-40 °C erwärmt. Sodann werden die Pellets bei einer Sprührate von 5 g/min und einem Sprühluftdruck von 2,5 bar mit einer 10 %igen wässrigen Hydroxypropylmethylcellulose-Lösung besprüht, wobei im Verlauf des Prozesses die Sprührate auf 8,5 g/min erhöht wird. Nach dem Aufbringen von 2215 g der Sprühlösung, entsprechend einem 14 %igen Feststoffauftrag, wird bei einer Produkttemperatur von max. 51 °C (Zulufttemperatur = etwa 70 °C) erneut getrocknet, anschließend auf etwa 27 °C abgekühlt und gesiebt.
[Alternativ können Trocknung und Coating bzw. Granulierung, Trocknung und Coating auch in einem gemeinsamen Arbeitsschritt erfolgen.]

**Beispiel 3: Herstellung von Farbstoffpellets im Top-Spray-Verfahren**

| Mischung A | |
|---|---|
| 721,6 g | Dextrose |

| Mischung B | (Dispersion) |
|---|---|
| 17,0 g | 2,5-Diamino-toluol-sulfat |
| 2,0 g | Resorcin |
| 7,6 g | 2-Methylresorcin |
| 2,2 g | 2-Amino-6-chlor-4-nitrophenol |
| 2,4 g | 6-Amino-m-kresol |
| 0,2 g | 4-Amino-2-hydroxytoluol |
| 3,0 g | Ascorbinsäure |
| 4,0 g | Natriumsulfit |
| 80,0 g | Alanin |
| 60,0 g | Glycin |
| 500,0 g | 20%ige wässrige Polyvinylpyrrolidon-Lösung |
| | (M = 30000 g/mol) |

Die Mischung (A) wird in der in Beispiel 1 beschriebenen Weise pelletiert, wobei jedoch als Sprühlösung die vorstehend beschriebene Mischung (B) verwendet wird.

**Beispiel 4: Oxidationshaarfärbemittel**

| Wasserstoffperoxidemulsion | |
|---|---|
| 9,00 g | Wasserstoffperoxid |
| 1,80 g | Cetylstearylalcohol |
| 3,30 g | Polyvinylpyrrolidon/Styrol-Copolymer |
| 0,20 g | Dinatriumphosphat |
| 0,20 g | Natriumlaurylsulfat |
| 0,10 g | Salicylsäure |

| | |
|---|---|
| 0,08 g | Phosphorsäure |
| ad 100,00 g | Wasser |

Die vorstehende Wasserstoffperoxidemulsion wird im klassischen Heiß-Emulgierverfahren hergestellt. Unmittelbar vor der Anwendung wird diese Wasserstoffperoxidemulsion mit Farbstoffpellets gemäß Beispiel 1, 2 oder 3 in einer Färbeschale oder Schüttelflasche vermischt.

Alle Prozentangaben stellen, soweit nicht anders angegeben, Gewichtsprozente dar.

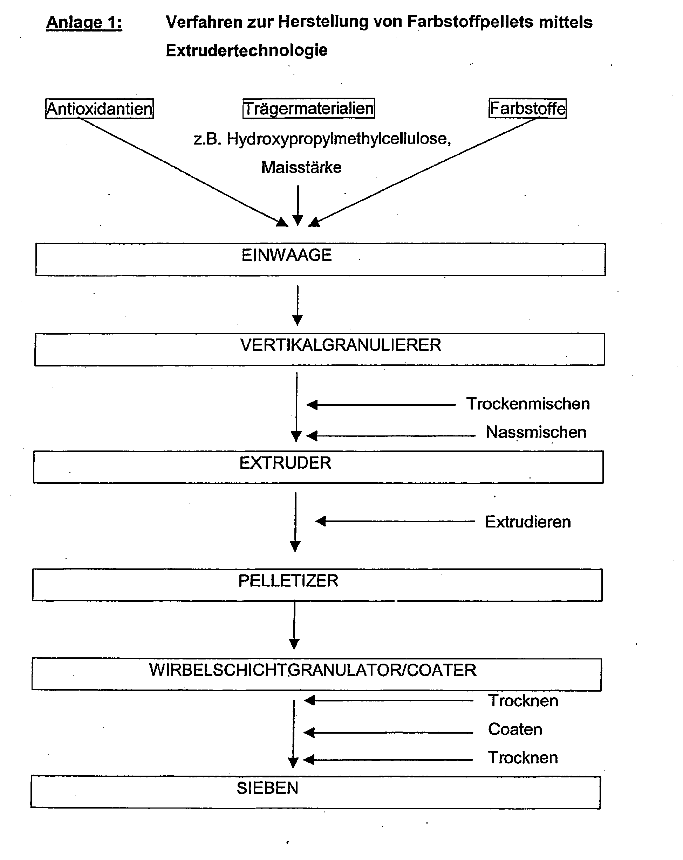

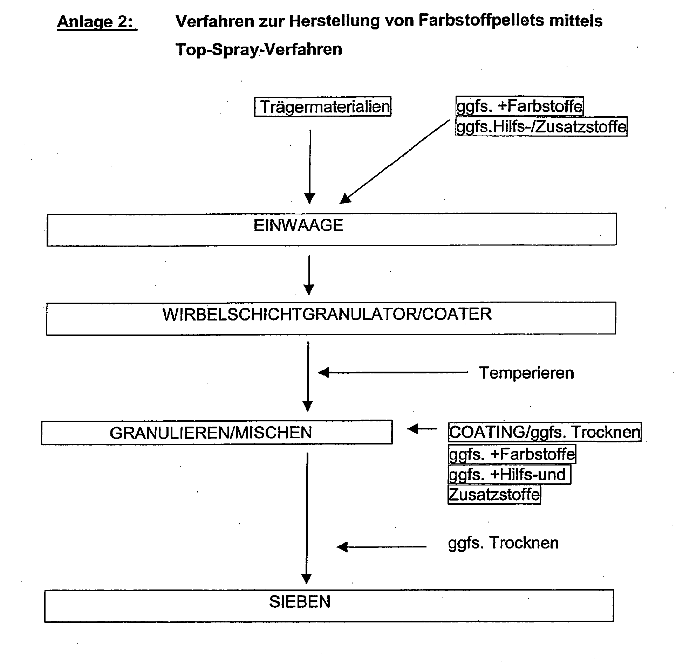

## Patentansprüche

1. Verfahren zur Herstellung farbstoff haltiger Pellets, **dadurch gekennzeichnet, dass** a) in einem Vertikalgranulierer bei einer Rotordrehzahl von 50 bis 200 U/min, einer Zerhackerdrehzahl von 750 bis 1250 U/min und einer Temperatur von 15 bis 35 °C durch Trockenmischen und anschließendes Nassmischen einer mindestens eine Oxidationsfarbstoffvorstufe enthaltenden Farbstoffmasse mit einem Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffen eine Grundmasse hergestellt wird; b) anschließend diese Grundmasse in einem Extruder bei einer Drehzahl von 15 bis 50 U/min und einer Lochgröße der Siebe von etwa 0,01 bis 5 mm extrudiert wird; c) das so erhaltene Granulat in einem Pelletizer bei einer Drehzahl von 400 bis 800 U/min verrundet wird; d) das Granulat sodann bei einer Produkttemperatur von 20 bis 60 °C und einer Zulufttemperatur von 70 bis 80 °C getrocknet wird; e) anschließend das Granulat bei einer Sprührate von 5 bis 20 g/min und einem Sprühluftdruck von 1,5 bis 2,5 bar im Wirbelschichtverfahren gecoatet wird; wobei das Verkapselungsmaterial, welches ausgewählt ist aus Cellulose-Derivaten, Polyethylen-Dispersionen, Polyacrylsäuren, Polyvinylalkoholen, Polyvinylpyrrplidonen, Polycarbonaten, Polyestern, Polyamiden und natürlichen Filmbildnern, -bezogen auf die Menge des zu coatenden Granulates- in einer Menge von 0,5 bis 50 Gewichtsprozent eingesetzt wird, und f) das Produkt abschließend bei einer Produkttemperatur von maximal 51 °C getrocknet wird.

2. Verfahren zur Herstellung farbstoffhaltiger Pellets, **dadurch gekennzeichnet, dass** in einem Wirbelschichtgranulierer a) bei einer Rotordrehzahl von 50 bis 200 U/min, einer Zerhackerdrehzahl von 750 bis 1250 U/min und einer Temperatur von 15 bis 35 °C eine mindestens eine Oxidationsfarbstoffvorstufe enthaltende Farbstoffmasse mit einem Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffen vermischt wird, anschließend auf eine Temperatur von max. 34 °C temperiert und sodann granuliert wird; b) das erhaltene Granulat bei einer Sprührate von 6 bis 20 g/min und einem Sprühluftdruck von 0,25 bis 0,75 bar mit einem Verkapselungsmaterial, welches ausgewählt ist aus Cellulose-Derivaten, Polyethylen-Dispersionen, Polyacrylsäuren, Polyvinylalkoholen, Polyvinylpyrrplidonen, Polycarbonaten, Polyestern, Polyamiden und natürlichen Filmbildnern gecoatet wird, wobei das Verkapselungsmaterial -bezogen auf die Menge des zu coatenden Granulates- in einer Menge von 0,5 bis 50 Gewichtsprozent eingesetzt wird; und c) falls erforderlich, das erhaltene Produkt bei einer Produkttemperatur von max. 60 °C getrocknet wird.

3. Verfahren zur Herstellung farbstoffhaltiger Pellets, **dadurch gekennzeichnet, dass** in einem Wirbelschichtgranulierer a) bei einer Rotordrehzahl von 50 bis 200 U/min, einer Zerhackerdrehzahl von 750 bis 1250 U/min und einer Temperatur von 15 bis 35 °C das Trägermaterial, welches ausgewählt ist aus Polyvinylpyrrolidonen, Dextrose, Oligosacchariden, mikrokristallinen Cellulose-Derivaten, physikalisch oder chemisch modifizierten Stärken oder Stärkederivaten, synthetischem Calciumsilicat, Kieselgur und Siliziumdioxid, sowie gegebenenfalls Antioxidantien und Hilfsstoffe miteinander vermischt werden; b) die so erhaltene Grundmasse auf eine Temperatur von max. 34 °C temperiert und granuliert wird; c) sodann bei einer Sprührate von 6 bis 20 g/min und einem Sprühluftdruck von 0,25 bis 0,75 bar mit einer Lösung oder Dispersion der mindestens eine Oxidationsfarbstoffvorstufe enthaltenden Farbstoffmasse in einem Verkapselungsmaterial, welches ausgewählt ist aus Cellulose-Derivaten, Polyethylen-Dispersionen, Polyacrylsäuren, Polyvinylalkoholen, Polyvinylpyrrplidonen, Polycarbonaten, Polyestern, Polyamiden und natürlichen Filmbildnern, gecoatet wird, wobei das Verkapselungsmaterial - bezogen auf die Menge des zu coatenden Granulates- in einer Menge von 0,5 bis 50 Gewichtsprozent eingesetzt wird; und d) falls erforderlich, das erhaltene Produkt bei einer Produkttemperatur von max. 57 °C getrocknet wird.

4. Verfahren nach einem der Ansprüche 1 bis 3, **dadurch gekennzeichnet, dass** das mikrokristalline Cellulose-Derivat und die modifizierten Stärken oder Stärkederivate ausgewählt sind aus Hydroxypropylmethylcellulose, Methylcellulose, Hydroxyethylcellulose, Hydroxypropylcellulose, Nonoxynol-Hydroxyethylcellulose und Cetyl-Hydroxyethylcellulose und mit Propylenoxid modifizierten nichtionischen Stärkederivaten.

5. Verfahren nach einem der Ansprüche 1 bis 3, **dadurch gekennzeichnet, dass** der natürliche Filmbildner ausgewählt ist aus Chitosan, Schellack, Oligosacchariden und chinesischem Balsamharz.

6. Verfahren nach einem der Ansprüche 1 bis 5, **dadurch gekennzeichnet, dass** die Oxidationsfarbstoffvorstufe ausgewählt ist aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamiho-1-(1-methylethyl)-1H-pyrazol, 4,5-Diamino-1-[(4-methylphenyl)methyl]-1H-pyrazol, 1-[(4-Chlorphenyl)methyl]-4,5-diamino-1 H-pyrazol, 4,5-Diamino-1-methyl-1H-pyrazol, 2-Amino-phenol, 2-Amino-6-methyl-phenol, 2-Amino-5-methyl-phenol, N-(3-Dimethylaminophenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-ethyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4₋methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, 2-Amino-5-methylphenol, 2-Amino-6-methylphenol, 2-Amino-5-ethoxyphenol und 2-Propyl-amino-5-aminopyridin.

7. Verfahren nach einem der Ansprüche 1 bis 6, **dadurch gekennzeichnet, dass** er zusätzlich einen natürlichen und/oder synthetischen direkt-ziehenden Farbstoff enthält.

8. Verfahren nach Anspruch 7, **dadurch gekennzeichnet, dass** der direktziehende Farbstoff ausgewählt ist aus Hydroxyethyl-2-nitro-p-toluidin, 2-Hydroxyethyl-pikraminsäure, 4-Nitrophenyl-aminoharnstoff, Basic Violet 2, Disperse Violet 1, HC Blue No. 2, HC Blue No. 12, HC Red No. 13, HC Red No. 3, 4-Amino-3-nitrophenol, 4-[(2-Hydroxyethyl)-amino]-3-nitrophenol, HC Red No. 10, HC Red No. 11, 2-Chlor-6-ethylamino-4-nitrophenol, 2-Amino-6-chlor-4-nitrophenol, HC Yellow No. 13, Basic Blue No. 99, Basic Brown No. 16, Basic Brown No. 17, Basic Red No. 76, Basic Yellow No. 57 und 2,6-Diamino-3-[(pyridin-3-yl)azo]-pyridin sowie deren Salzen.

9. Mittel zur Färbung von Keratinfasern, erhalten durch Vermischen mindestens eines gemäß dem Verfahren nach einem der Ansprüche 1 bis 8 hergestellten farbstoffhaltigen Pellets mit einer wässrigen oder wässrigalkohlischen Zubereitung.

10. Mittel nach Anspruch 9, **dadurch gekennzeichnet, dass** die wässrige oder wässrig-alkohlische Zubereitung ausgewählt ist aus Wasser, Gemischen aus Wasser und C1-C6-Alkoholen, Wasserstoffperoxidlösungen oder Wasserstoffperoxidemulsionen, Haarreinigungsmitteln, Haarkonditionierungsmitteln und Haarfestigungsmittel.

11. Verwendung eines gemäß dem Verfahren nach einem der Ansprüche 1 bis 8 hergestellten farbstoffhaltigen Pellets zur Herstellung eines Färbemittels für Keratinfasern.

## Claims

1. Process for the preparation of dye-containing pellets, **characterized in that** a) a basic mass is prepared in a vertical granulator at a rotor speed of from 50 to 200 rpm, a chopper speed of from 750 to 1250 rpm and a temperature of from 15 to 35°C by dry-mixing and subsequent wet-mixing a dye mass comprising at least one oxidation dye precursor with a carrier material which is selected from polyvinylpyrrolidones, dextrose, oligosaccharides, microcrystalline cellulose derivatives, physically or chemically modified starches or starch derivatives, synthetic calcium silicate, kieselguhr and silicon dioxide, and optionally antioxidants and auxiliaries; b) then this basic mass is extruded in an extruder at a speed of from 15 to 50 rpm and a hole size in the sieve of from about 0.01 to 5 mm; c) the granules obtained in this way are rounded in a pelletizer at a speed of from 400 to 800 rpm; d) the granules are then dried at a product temperature of from 20 to 60°C and an incoming air temperature of from 70 to 80°C; e) then the granules are coated at a spraying rate of from 5 to 20 g/min and a spraying air pressure of from 1.5 to 2.5 bar in a fluidized-bed method, where the encapsulation material, which is selected from cellulose derivatives, polyethylene dispersions, polyacrylic acids, polyvinyl alcohols, polyvinylpyrrolidones, polycarbonates, polyesters, polyamides and natural film formers, is used in an amount of from 0.5 to 50 percent by weight, based on the amount of the granules to be coated, and f) the product is finally dried at a product temperature of at most 51°C.

2. Process for the preparation of dye-containing pellets, **characterized in that**, in a fluidized-bed granulator a) at a rotor speed of from 50 to 200 rpm, a chopper speed of from 750 to 1250 rpm and a temperature of from 15 to 35°C, a dye mass comprising at least one oxidation dye precursor is mixed with a carrier material which is selected from polyvinylpyrrolidones, dextrose, oligosaccharides, microcrystalline cellulose derivatives, physically or chemically modified starches or starch derivatives, synthetic calcium silicate, kieselguhr and silicon dioxide, and optionally antioxidants and auxiliaries, then heated to a temperature of at most 34°C and then granulated; b) the resulting granules are coated at a spraying rate of from 6 to 20 g/min and a spraying air pressure of from 0.25 to 0.75 bar with an encapsulation material which is selected from cellulose derivatives, polyethylene dispersions, polyacrylic acids, polyvinyl alcohols, polyvinylpyrrolidones, polycarbonates, polyesters, polyamides and natural film formers, where the encapsulation material is used in an amount of from 0.5 to 50 percent by weight, based on the amount of the granules to be coated; and c) if required, the resulting product is dried at a product temperature of at most 60°C.

3. Process for the preparation of dye-containing pellets, **characterized in that**, in a fluidized-bed granulator a) at a rotor speed of from 50 to 200 rpm, a chopper speed of from 750 to 1250 rpm and a temperature of from 15 to 35°C, the carrier material, which is selected from polyvinylpyrrolidones, dextrose, oligosaccharides, microcrystalline cellulose derivatives, physically or chemically modified starches or starch derivatives, synthetic calcium silicate, kieselguhr and silicon dioxide, and also optionally antioxidants and auxiliaries are mixed together; b) the basic mass obtained in this way is heated to a temperature of at most 34°C and granulated; c) then is coated at a spraying rate of from 6 to 20 g/min and a spraying air pressure of from 0.25 to 0.75 bar with a solution or dispersion of the dye mass comprising at least one oxidation dye precursor in an encapsulation material which is selected from cellulose derivatives, polyethylene dispersions, polyacrylic acids, polyvinyl alcohols, polyvinylpyrrolidones, polycarbonates, polyesters, polyamides and natural film formers, where the encapsulation material is used in an amount of from 0.5 to 50 percent by weight, based on the amount of the granules to be coated; and d) if required, the resulting product is dried at a product temperature of at most 57°C.

4. Process according to one of Claims 1 to 3, **characterized in that** the microcrystalline cellulose derivative and the modified starches or starch derivatives are selected from hydroxypropylmethylcellulose, methylcellulose, hydroxyethylcellulose, hydroxypropylcellulose, nonoxynolhydroxyethylcellulose and cetylhydroxyethylcellulose and nonionic starch derivatives modified with propylene oxide.

5. Process according to one of Claims 1 to 3, **characterized in that** the natural film former is selected from chitosan, shellac, oligosaccharides and Chinese balsam resin.

6. Process according to one of Claims 1 to 5, **characterized in that** the oxidation dye precursor is selected from 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-acetylamino)-ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)-amino]aniline, 4-[di(2-hydroxyethyl)amino]aniline, 4-[di(2-hydroxyethyl)amino]-2-methylaniline, 4-[(2-methoxyethyl)amino]aniline, 4-[(3-hydroxypropyl)amino]aniline, 4-[(2,3-dihydroxypropyl)-amino]aniline, 1,4-diamino-2-(2-hydroxyethyl)-benzene, 1,4-diamino-2-(1-methylethyl)benzene, 1,3-bis[(4-aminophenyl)(2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)-phenol, 4-amino-2-(hydroxymethyl)phenol, 4-amino-2-fluorophenol, 4-amino-2-[(2-hydroxyethyl)amino]-methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)-methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol, N-(3-dimethylaminophenyl)-urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]-aniline, 1,3-di(2,4-diaminophenoxy)propane, di(2,4-diaminophenoxy)methane, 1,3-diamino-2,4-dimethoxybenzene, 2,6-bis(2-hydroxyethyl)-aminotoluene, 4-hydroxyindole, 3-dimethylamino-phenol, 3-diethylaminophenol, 5-amino-2-methyl-phenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methyl-phenol, 3-amino-2-chloro-6-methylphenol, 3-aminophenol, 2-[(3-hydroxyphenyl)amino]acetamide, 5-[(2-hydroxyethyl)amino]-4-methoxy-2-methyl-phenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 2-methyl-l-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-l-phenyl-5-pyrazolone, 5,6-dihydroxyindole, 5,6-dihydroxyindoline, 5-hydroxyindole, 6-hydroxyindole, 7-hydroxyindole, 2,3-indolinedione, 2-amino-5-methylphenol, 2-amino-6-methylphenol, 2-amino-5-ethoxyphenol and 2-propylamino-5-aminopyridine.

7. Process according to one of Claims 1 to 6, **characterized in that** it additionally comprises a natural and/or synthetic direct dye.

8. Process according to Claim 7, **characterized in that** the direct dye is selected from hydroxyethyl-2-nitro-p-toluidine, 2-hydroxyethylpicramic acid, 4-nitrophenylaminourea, Basic Violet 2, Disperse Violet 1, HC Blue No. 2, HC Blue No. 12, HC Red No. 13, HC Red No. 3, 4-amino-3-nitrophenol, 4-[(2-hydroxyethyl)amino]3-nitrophenol, HC Red No. 10, HC Red No. 11, 2-chloro-6-ethylamino-4-nitrophenol, 2-amino-6-chloro-4-nitrophenol, HC Yellow No. 13, Basic Blue No. 99, Basic Brown No. 16, Basic Brown No. 17, Basic Red No. 76, Basic Yellow No. 57 and 2,6-diamino-3-[(pyridin-3-yl)azo]pyridine and salts thereof.

9. Agent for dyeing keratin fibres, obtained by mixing at least one dye-containing pellet prepared by the process according to one of Claims 1 to 8 with an aqueous or aqueous-alcoholic preparation.

10. Agent according to Claim 9, **characterized in that** the aqueous or aqueous-alcoholic preparation is selected from water, mixtures of water and C1-C6-alcohols, hydrogen peroxide solutions or hydrogen peroxide emulsions, hair cleansing compositions, hair conditioning compositions and hair setting compositions.

11. The use of a dye-containing pellet prepared by the process according to one of Claims 1 to 8 for the preparation of a colorant for keratin fibres.

## Revendications

1. Procédé pour la production de granules contenant un colorant, **caractérisé en ce que** a) dans un granulateur vertical, à une vitesse du rotor de 50 à 200 tours/min, une vitesse du hacheur de 750 à 1 250 tours/min et à une température de 15 à 35 °C, on prépare une matière de base par mélange à sec et mélange subséquent par voie humide d'une matière colorante contenant au moins un précurseur de colorant d'oxydation avec un matériau de support qui est choisi parmi les polyvinylpyrrolidones, le D-glucose, les oligosaccharides, les dérivés de cellulose microcristallins, les amidons ou dérivés d'amidon modifiés physiquement ou chimiquement, le silicate de calcium synthétique, le kieselguhr et le dioxyde de silicium, ainsi qu'éventuellement des antioxydants et adjuvants ; b) cette matière de base est ensuite extrudée dans une extrudeuse à une vitesse de rotation de 15 à 50 tours/min et à une taille de trous des tamis d'environ 0,01 à 5 mm ; c) le produit granulé ainsi obtenu est arrondi dans une pastilleuse, à une vitesse de rotation de 400 à 800 tours/min ; i d) le produit granulé est ensuite séché à une température du produit de 20 à 60 °C et à une température de l'air entrant de 70 à 80°C ; e) le granulé est ensuite enrobé dans le procédé en lit tourbillonnaire, à une vitesse de pulvérisation de 5 à 20 g/min et sous une pression d'air de pulvérisation de 1,5 à 2,5 bars, le matériau d'encapsulation qui est choisi parmi les dérivés de cellulose, des dispersions de polyéthylène, les poly(acide acrylique)s, les poly(alcool vinylique)s, les polyvinylpyrrolidones, les polycarbonates, les polyesters, les polyamides et des agents filmogènes naturels, étant utilisé en une quantité de 0,5 à 50 % en poids - par rapport à la quantité du produit granulé à enrober - et f) le produit est ensuite séché à une température du produit de 51 °C au maximum.

2. Procédé pour la production de granules contenant un colorant, **caractérisé en ce que** dans un granulateur à lit tourbillonnaire a) à une vitesse du rotor de 50 à 200 tours/min, une vitesse du hacheur de 750 à 1 250 tours/min et à une température de 15 à 35 °C, on mélange une matière de base contenant au moins un précurseur de colorant d'oxydation avec un matériau de support qui est choisi parmi les polyvinylpyrrolidones, le D-glucose, les oligosaccharides, les dérivés de cellulose microcristallins, les amidons ou dérivés d'amidon modifiés physiquement ou chimiquement, le silicate de calcium synthétique, le kieselguhr et le dioxyde de silicium, ainsi qu'éventuellement des antioxydants et adjuvants, puis on la traite thermiquement à une température d'au maximum 34 °C et ensuite on la granule ; b) le produit granulé obtenu est enrobé, à une vitesse de pulvérisation de 6 à 20 g/min et sous une pression d'air de pulvérisation de 0,25 à 0,75 bar, avec un matériau d'encapsulation qui est choisi parmi les dérivés de cellulose, des dispersions de polyéthylène, les poly(acide acrylique)s, les poly(alcool vinylique)s, les polyvinylpyrrolidones, les polycarbonates, les polyesters, les polyamides et des agents filmogènes naturels, le matériau d'encapsulation étant utilisé en une quantité de 0,5 à 50 % en poids - par rapport à la quantité du produit granulé à enrober - et c) si nécessaire, le produit obtenu est séché à une température du produit de 60 °C au maximum.

3. Procédé pour la production de granules contenant un colorant, **caractérisé en ce que** dans un granulateur à lit tourbillonnaire a) à une vitesse du rotor de 50 à 200 tours/min, une vitesse du hacheur de 750 à 1 250 tours/min et à une température de 15 à 35 °C, on mélange entre eux le matériau de support qui est choisi parmi les polyvinylpyrrolidones, le D-glucose, les oligosaccharides, les dérivés de cellulose microcristallins, les amidons ou dérivés d'amidon modifiés physiquement ou chimiquement, le silicate de calcium synthétique, le kieselguhr et le dioxyde de silicium, ainsi qu'éventuellement des antioxydants et adjuvants ; b) la matière de base ainsi obtenue est traitée thermiquement à une température d'au maximum 34 °C et granulée ; i c) ensuite enrobée, à une vitesse de pulvérisation de 6 à 20 g/min et. sous une pression d'air de pulvérisation de 0,25 à 0,75 bar, avec une solution ou dispersion de la matière colorante contenant au moins un précurseur de colorant d'oxydation dans un matériau d'encapsulation qui est choisi parmi les dérivés de cellulose, des dispersions de polyéthylène, les poly(acide acrylique)s, les poly(alcool vinylique)s, les polyvinylpyrrolidones, les polycarbonates, les polyesters, les polyamides et des agents filmogènes naturels, le matériau d'encapsulation étant utilisé en une quantité de 0,5 à 50 % en poids - par rapport à la quantité du produit granulé à enrober - et d) si nécessaire, le produit obtenu est séché à une température du produit de 57 °C au maximum.

4. Procédé selon l'une quelconque des revendications 1 à 3, **caractérisé en ce que** le dérivé de cellulose microcristalline et les amidons ou dérivés d'amidon modifiés sont choisis parmi l'hydroxypropylméthylcellulose, la méthylcellulose, l'hydroxyéthylcellulose, l'hydroxypropylcellulose, la nonoxynol-hydroxyéthylcellulose et la cétylhydroxyéthylcellulose et des dérivés d'amidon non ioniques modifiés avec de l'oxyde de propylène.

5. Procédé selon l'une quelconque des revendications 1 à 3, **caractérisé en ce que** l'agent filmogène naturel est choisi parmi le chitosane, la gomme-laque, des oligosaccharides et la résine de baume chinois.

6. Procédé selon l'une quelconque des revendications 1 à 5, **caractérisé en ce que** le précurseur de colorant d'oxydation est choisi parmi le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)benzène, le 2,5-diaminobiphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)benzène, le 2-(2-(acétylamino)-éthoxy)-1,4-diaminobenzène, la 4-phénylaminoaniline, la 4-diméthylamino-aniline, la 4-diéthylamino-aniline, la 4-dipropylaminoaniline, la 4-[éthyl-(2-hydroxyéthyl)amino]-aniline, la 4-[di(2-hydroxyéthyl)amino]aniline, la 4-[di(2-hydroxyéthyl)amino]-2-méthylaniline, la 4-[(2-méthoxyéthyl)amino]aniline, la 4-[(3-hydroxypropyl)amino] aniline, la 4-[(2,3-dihydroxypropyl)amino]aniline, le 1,4-diamino-2-(2-hydroxyéthyl)benzène, le 1,4-diamino-2-(1-méthyléthyl)-benzène, le 1,3-bis[(4-aminophényl)(2-hydroxyéthyl)amino]-2-propanol, le 1,4-bis[(4-aminophényl)amino]butane, le 1,8-bis(2,5-diaminophénoxy)-3,6-dioxaoctane, le 4-aminophénol, le 4-amino-3-méthylphénol, le 4-amino-3-(hydroxyméthyl)phénol, le 4-amino-3-fluoro-phénol, le 4-méthylamino-phénol, le 4-amino-2-(aminométhyl)phénol, le 4-amino-2-(hydroxyméthyl)phénol, le 4-amino-2-fluoro-phénol, le 4-amino-2-[(2-hydroxyéthyl)amino]méthylphénol, le 4-amino-2-méthyl-phénol, le 4-amino-2-(méthoxyméthyl)phénol, le 4-amino-2-(2-hydroxyéthyl)phénol, l'acide 5-aminosalicylique, la 2,5-diaminopyridine, le 2,4,5,6-tétraaminopyrimidine, le 2,5,6-triamino-4- . (1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)méthyl]-4,5-diamino-1H-pyrazole, le 4,5-diamino-1-méthyl-1H-pyrazole, le 2-aminophénol, le 2-amino-6-méthylphénol, le 2-amino-5-méthylphénol, la N-(3-diméthylaminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 1,3-diamino-4-(2,3-dihydroxypropoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]-aniline, le 4-amino-2-di[(2-hydroxyéthyl)amino]-1-éthoxybenzène, le 5-méthyl-2-(1-méthyléthyl)-phénol, la 3-[(2-hydroxyéthyl)amino]aniline, la 3-[(2-aminoéthyl)amino]aniline, le 1,3-di(2,4-diaminophënoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]-acétamide, le 5-[(2-hydroxyéthyl)amino]-4-méthoxy-2-méthyl-phénol, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 5-amino-2-méthoxy-phénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthyl-phénol, la 2-amino-3-hydroxypyridine, le 5-amino-4-chloro-2-méthylphénol, le 1-naphtol, le 2-méthyl-1-naphtol, le 1,5-dihydroxynaphthalène, le 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 5-hydroxy-indole, le 6-hydroxy-indole, le 7-hydroxy-indole, la 2,3-indolinedione, le 2-amino-5-méthylphénol, le 2-amino-6-méthylphénol, le 2-amino-5-éthoxyphénol et la 2-propyl-amino-5-aminopyridine.

7. Procédé selon l'une quelconque des revendications 1 à 6, **caractérisé en ce que** la matière colorante contient en outre un colorant direct naturel et/ou synthétique.

8. Procédé selon la revendication 7, **caractérisé en ce que** le colorant direct est choisi parmi l'hydroxyéthyl-2-nitro-p-toluidine, l'acide 2-hydroxyéthyl-picramique, la 4-nitrophénylamino-urée, le Basic Violet 2, le Disperse Violet 1, le HC Blue n° 2, le HC Blue n0 12, le HC Red n° 13, le HC Red n° 3, le 4-amino-3-nitrophénol, le 4-[(2-hydroxyéthyl)-amino]-3-nitrophénol, le HC Red n° 10, le HC Red n° 11, le 2-chloro-6-éthylamino-4-nitrophénol, le 2-amino-6-chloro-4-nitrophénol, le HC Yellow n° 13, le Basic Blue n° 99, le Basic Brown n° 16, le Basic Brown n° 17, le Basic Red n° 76, le Basic Yellow n° 57 et la 2,6-diamino-3-[(pyridin-3-yl)azo]pyridine ainsi que leurs sels.

9. Composition pour la teinture de fibres de kératine, obtenue par mélange d'au moins un granule contenant un colorant, produit conformément au procédé selon l'une quelconque des revendications 1 à 8, avec une préparation aqueuse ou aqueuse-alcoolique.

10. Composition selon la revendication 9, **caractérisée en ce que** la préparation aqueuse ou aqueuse-alcoolique est choisie parmi l'eau, des mélanges d'eau et d'alcools en C₁-C₆, des solutions de peroxyde d'hydrogène ou des émulsions de peroxyde d'hydrogène, des produits pour le nettoyage des cheveux, des conditionneurs pour cheveux et des fixateurs pour cheveux.

11. Utilisation d'un granule contenant un colorant, produit conformément au procédé selon l'une quelconque des revendications 1 à 8, pour la préparation d'une composition de teinture pour fibres de kératine.
